(19) Europäisches Patentamt

European Patent Office

Office européen des brevets

(11) Publication number: **0 555 553 A2**

# EUROPEAN PATENT APPLICATION

(21) Application number: **92121847.5**

(22) Date of filing: **23.12.92**

(51) Int. Cl.5: **A61B 5/00**

(30) Priority: **07.02.92 US 832551**

(43) Date of publication of application:
**18.08.93 Bulletin 93/33**

(84) Designated Contracting States:
**DE FR GB IT**

(71) Applicant: **BOC Health Care, Inc.**
**575 Mountain Avenue, Murray Hill**
**New Providence, New Jersey 07974(US)**

(72) Inventor: **Pologe, Jonas Alexander**
**515 Hartford Drive**
**Boulder, Colorado 80303(US)**

(74) Representative: **Dipl.-Phys.Dr. Manitz**
**Dipl.-Ing.Dipl.-Wirtsch.-Ing. Finsterwald**
**Dipl.-Phys. Rotermund Dipl.-Chem.Dr. Heyn**
**B.Sc.(Phys.) Morgan**
**Postfach 22 16 11**
**D-80506 München (DE)**

(54) **Improved arterial blood monitoring system.**

(57) This improved arterial blood monitoring system takes advantage of the basic statistical property of arterial blood that arterial blood contains a plurality of dominant absorbers, whose measured light absorption spectra appear as a constant over a short interval of time. The arterial blood characteristics to be measured are empirically related to the changes in the measured light transmission through the plurality of dominant absorbers as a function of the change in arterial blood volume at the probe site. Therefore, by measuring the transmitted light as it varies with arterial pulsation at selected wavelengths of light, the relative amount of these dominant absorbers in the arterial blood can noninvasively be determined. By selecting one wavelength of light around 1270nm, where water has a measurable extinction and a second wavelength at about 810nm, a wavelength that is substantially isobestic to oxygenated and reduced hemoglobin, a direct relationship between the transmitted intensities at these two wavelengths and the arterial hemoglobin concentration will exist and can be calculated. The use of a plurality of wavelengths of light enable the arterial blood monitoring system to concurrently measure a number of characteristics of arterial blood. By selecting wavelengths of light around 660nm, 940nm and 1270nm, the values of total hemoglobin and oxygen saturation of arterial blood can simultaneously be determined. In addition, oxygen content of arterial blood can be calculated from these values of total hemoglobin and oxygen saturation.

EP 0 555 553 A2

Rank Xerox (UK) Business Services
(3. 10/3.6/3.3. 1)

## FIELD OF THE INVENTION

This invention relates to pulse oximeters which noninvasively measure the oxygen saturation of hemoglobin in arterial blood and, in particular, to an improved arterial blood monitoring system for performing these calculations and for also measuring a number of additional parameters, including: total hemoglobin and oxygen content of arterial blood.

## PROBLEM

It is a problem in the field medical monitoring equipment to accurately measure various parameters of arterial blood in a noninvasive manner. For example, the oxygen saturation ($S_aO_2$) of the hemoglobin in arterial blood is determined by the relative proportions of oxygenated hemoglobin and reduced hemoglobin in the arterial blood. A pulse oximeter system noninvasively determines the oxygen saturation of the hemoglobin by measuring the difference in the light absorption of these two forms of hemoglobin. Reduced hemoglobin absorbs more light in the red band than does oxyhemoglobin while oxyhemoglobin absorbs more light in the near infrared band (800 - 1000nm) than does reduced hemoglobin. The pulse oximeter includes a probe that is placed in contact with the skin, either on a flat surface in the case of reflectance probes or across some appendage in the transmission case. The probe contains two light emitting diodes, each of which emits light at a specific wavelength, one in the red band and one in the infrared band. The amount of light transmitted through the intervening appendage is measured many times a second at both wavelengths.

The appendage contains arterial, capillary and venous blood as well as intervening tissue and bone. Therefore the red and infrared signals received from the probe contain a non-pulsatile component which is influenced by the absorbency of tissue, venous blood, capillary blood, non-pulsatile arterial blood, the intensity of the light source and the sensitivity of the detector. The pulsatile component of the received signals is an indication of the expansion of the arteriolar bed with arterial blood. The amplitude of the pulsatile component is a very small percentage of the total signal amplitude and depends on the blood volume per pulse and the $S_aO_2$. The received red and infrared signals have an exponential relationship to the path length of the arterial blood. The effects of different tissue thicknesses and skin pigmentation can be removed from the received signals by normalizing the received signal by a term that is proportional to the non-pulsatile incident intensity. Taking the ratio of the mathematically processed and normalized red and infrared signals results in a number which is theoretically a function of only the concentration of oxyhemoglobin and reduced hemoglobin in the arterial blood, provided that the concentration of dyshemoglobins in the arterial blood is sufficiently small.

Measuring $S_aO_2$ alone provides an incomplete picture of patient oxygenation status. To help complete our knowledge of oxygen delivery, we need to know, not only the percentage of hemoglobin bound to oxygen but also just how much hemoglobin is available. There presently does not exist any medical monitoring equipment, comparable to a pulse oximeter system, for noninvasively measuring other parameters of arterial blood, such as total hemoglobin content and oxygen content. These parameters are either measured in an infrequent invasive manner or not directly measured at all. It is therefore a problem in the field of medical monitoring equipment to concurrently measure in real time a plurality of characteristics of arterial blood in a noninvasive manner. It is also desirable to minimize the number of pieces of equipment required to perform these measurements as well as the number of probes required to collect the data.

## SOLUTION

The above described problems are solved and a technical advance achieved in the field by the improved arterial blood monitoring system, that allows for noninvasively measuring and calculating a plurality of characteristics of arterial blood. This improved arterial blood monitoring system takes advantage of the basic statistical property of arterial blood that arterial blood contains a plurality of dominant absorbers, whose measured light absorption spectra appear as a constant over a short interval of time. The arterial blood characteristics to be measured are empirically related to the changes in the measured light transmission through the plurality of dominant absorbers as a function of the changes in arterial blood volume at the probe site. By measuring the transmitted light as it varies with arterial pulsation at selected wavelengths of light, the relative amount of these dominant absorbers in the arterial blood can noninvasively be determined. The wavelengths of light are selected such that the dominant absorbers have different extinction coefficients at at least one of these wavelengths of light. The wavelengths can also be selected to be substantially isobestic to a number of the dominant absorbers or their components.

Arterial blood contains a number of dominant absorbers, including water and hemoglobin. The hemoglobin itself is comprised of oxygenated hemoglobin, deoxygenated hemoglobin, carboxyhemoglobin, etc. The concentration of n of these components can be determined by selecting n different wavelengths of light where each of the n components have different extinction coefficients at at least one of the wavelengths of light. In addition, in a two wavelength system, one of the wavelengths of light is selected such that the two primary components of hemoglobin, oxygenated hemoglobin and deoxygenated hemoglobin, are substantially isobestic at that wavelength of light. By selecting one wavelength of light around 1270nm, where water has a measurable extinction and a second wavelength at about 810nm, a wavelength that is substantially isobestic to oxygenated and reduced hemoglobin, a direct relationship between the transmitted intensities at these two wavelengths and the arterial hemoglobin concentration will exist and can be calculated.

The use of a plurality of wavelengths of light enable the arterial blood monitoring system to concurrently measure a number of characteristics of arterial blood. By selecting wavelengths of light around 660nm, 940nm and 1270nm, the values of total hemoglobin and oxygen saturation of arterial blood can simultaneously be determined. In addition, oxygen content of arterial blood can be calculated from these values of total hemoglobin and oxygen saturation. Thus, a plurality of characteristics of arterial blood can be noninvasively determined by the arterial blood monitoring system. This system is able to measure this particular plurality of characteristics of arterial blood as a result of extending the techniques of pulse oximetry to include measurements at a wavelength where water has a measurable extinction. The use of a single probe and multiple wavelengths of light simplifies the apparatus required to perform the desired measurements and solves the problems of the prior art.

## BRIEF DESCRIPTION OF THE DRAWING

Figure 1 illustrates in block diagram form the overall architecture of the improved arterial blood monitoring system;

Figure 2 illustrates a typical display for the improved arterial blood monitoring system;

Figure 3 illustrates in graphical form the various components of the input signal from the probe; and

Figures 4 and 5 illustrate in flow chart form the operational steps taken by this system to determine the desired arterial blood characteristics.

## DETAILED DESCRIPTION

The improved arterial blood monitoring system takes advantage of the basic statistical property of arterial blood that arterial blood contains a plurality of dominant absorbers, whose measured light absorption spectra appear as a constant over a short interval of time. The arterial blood characteristics to be measured are empirically related to the changes in the measured light transmission through the plurality of dominant absorbers as a function of the changes in the arterial blood volume at the probe site. Therefore, by measuring the transmitted light as it varies with arterial pulsation, at selected wavelengths, the relative amount of these dominant absorbers in the arterial blood can noninvasively be determined. A single probe can be used to generate the plurality of wavelengths of light, therefore simplifying the arterial blood monitoring system.

## Definition of Terms

$I_0$ = The intensity of the beam of light at a given wavelength incident on the tissue-under-test, where the wavelength is denoted by the subscript.

I = The instantaneous value of the intensity of the light received by the detector. The light is at a given wavelength, which wavelength is indicated by a subscript.

$\epsilon$ = The extinction coefficient of light by a given substance (indicated by a superscript) at a given wavelength (indicated by a subscript).

C = The concentration of a given substance (indicated by a superscript).

L = The pathlength of a given substance (indicated by a superscript).

tHb = Total hemoglobin measured in arterial blood. Usually expressed in terms of grams per deciliter.

O = Used as a superscript to represent oxyhemoglobin.

R = Used as a superscript to represent reduced hemoglobin.

W = Used as a superscript to represent water.

t =       Used as a superscript to represent the combination of oxyhemoglobin and reduced hemoglobin.

## System Architecture

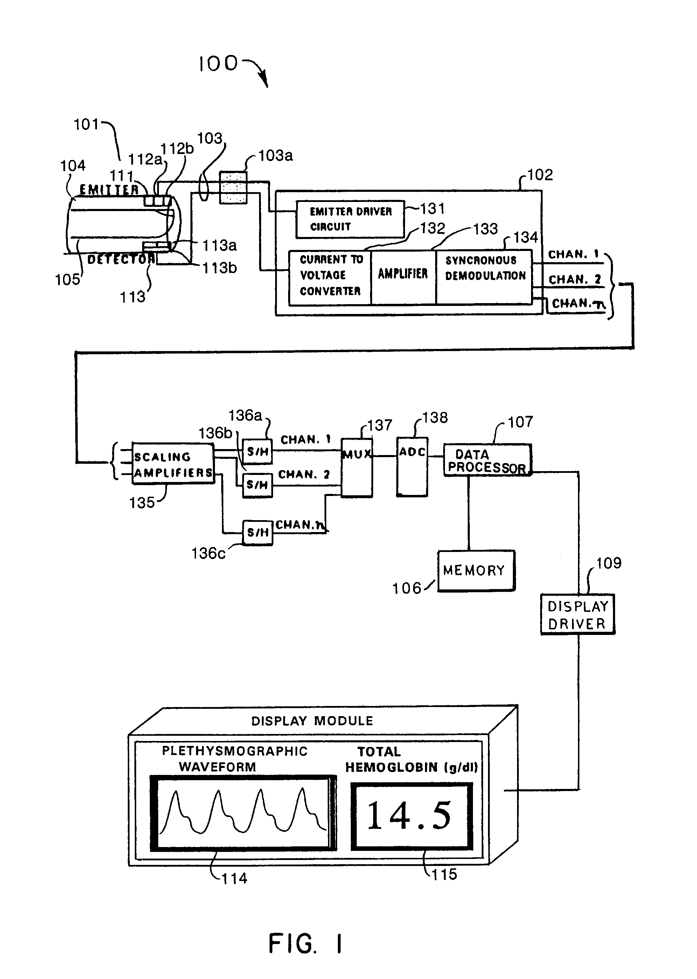

Figure 1 illustrates in block diagram form the overall architecture of the arterial blood monitoring system of the present invention. The arterial blood monitoring system 100 consists of a probe 101 connected to probe interface circuit 102 by means of a set of electrical conductors 103 and connector 103a. The probe 101 consists of an exterior housing 104 that applies the active elements of the probe to the tissue under test, such as a finger 105, containing an arterial blood flow that is to be monitored. Included within housing 104 is a plurality (at least two) of light emitting devices 111, 112 and at least one corresponding light detector 113. The light emitting devices 111, 112 each produce an output light beam of predetermined wavelength which is directed at the finger 105 enclosed by housing 104. The light detector 113 monitors the level of light that is transmitted through or reflected from finger 105. In order to distinguish between the light beams produced by first 111 and second 112 light emitting devices, these light emitting devices 111, 112 are modulated in a manner to allow the output of the light detector 113 to be synchronously demodulated. Ambient light, being unmodulated, is easily eliminated by the demodulator process.

The signals received by probe interface circuit 102 from light detector 113 are typically processed by additional analog circuitry 135 - 137 and then converted by an analog-to-digital converter circuit 138 into sets of digital measurements which are stored in memory 106. Data processing circuit 107 mathematically processes the digitized measurements stored in memory 106 to compute the desired characteristics based upon the measurements taken on the components in the arterial blood in finger 105. It is also possible that analog circuitry can be used to perform some of the mathematical operations described herein as performed by data processing circuit 107, such as taking derivatives or logarithms.

The results of the computations are displayed numerically via display driver 109 and the associated display 115 while the plethysmographic waveform is typically displayed graphically via display 114. The operation of data processing circuit 107 is disclosed in additional detail below and, for the purpose of this disclosure, it is assumed that many of the other elements disclosed in Figure 1 are the conventional components found in existing pulse oximeter systems.

## Probe

Probe 101 contains a minimum of two light emitting devices 111, 112, each of which produces a beam of light at a distinct wavelength. Probe 101 also contains light detector(s) 113 capable of receiving the emitted wavelengths of light. The beams of light produced at a specific wavelengths are referred to as $Io_1$, $Io_2$,...$Io_N$. while the received beams of light are referred to as $I_1$...$I_N$. In the present implementation, the light detector 113 consists of a multiple layer element that contains a germanium photodiode 113b placed under a silicon photodiode 113a. For the wavelengths of light shorter than approximately 1000nm, the silicon photodiode 113a receives the incident light. Above this wavelength, the silicon photodiode 113a becomes transparent and the germanium photodiode 113b picks up the incident light. Probe 101 includes a cable 103 and connector 103a for transmitting and receiving signals between probe 101 and probe interface circuit 102. Probe 101 is positioned on the tissue either in the transmission mode: light emitting devices 111, 112 on one side and light detector(s) 113 on the other side of finger 105, earlobe, toe or other appropriate site through which light can be received by the light detector(s) 113 at acceptable signal levels; or in the reflectance mode: in which the light emitting devices 111, 112 and light detector(s) 113 are placed on the same side of the tissue under test, such as the forehead or forearm.

## Signal Components

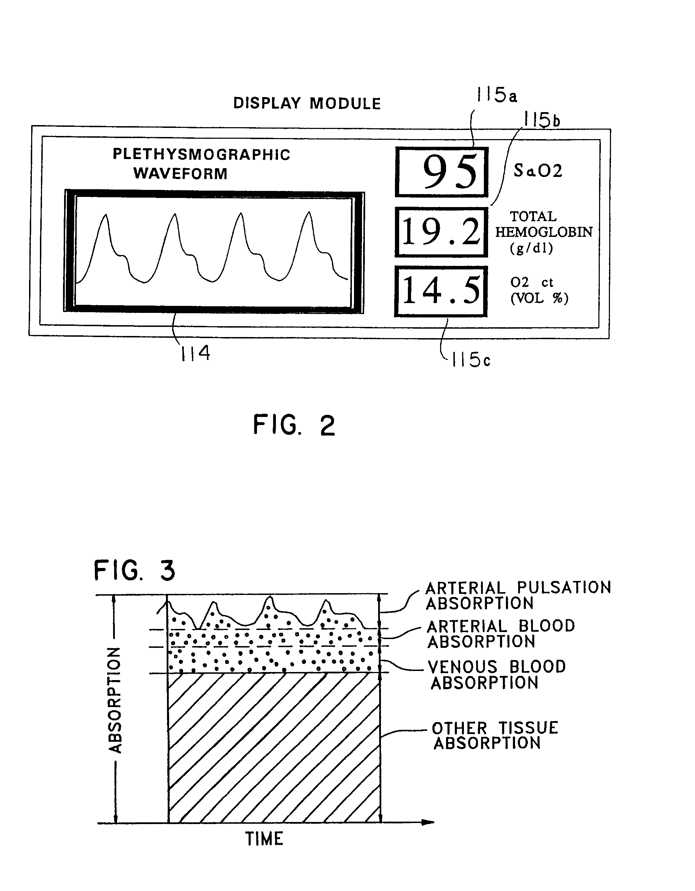

Figure 3 illustrates in graphical form (not to scale) the various components of the total absorption produced by the finger 105. The light detector output signal, high where absorption is low and visa versa, consists of a large magnitude non-pulsatile component and a small magnitude pulsatile component. The non-pulsatile component represents light remaining after absorption due to a combination of venous blood flow, cutaneous tissue, bone, and constant arterial blood flow while the small pulsatile component is caused by the light absorption due to pulsatile arterial blood flow that is to be measured. Following synchronous demodulation the data signals produced by the light detector 113 and transmitted to probe interface circuit 102 consist of a series of data points that are digitized and stored in memory 106. Since the first 111 and second 112 light emitting devices are sampled simultaneously and in rapid succession, these digitized data points consist of a plurality of sets of measurements, with one set corresponding to samples of the light beam intensity at a first wavelength, the other set corresponding to samples of the light beam intensity at a second wavelength, and, in some schemes, a third set corresponding to the intensity of the ambient light.

Ideally, in pulse oximeter systems red and infrared wavelengths of light are used and the ratio of the normalized derivative (or logarithm) of the red intensity to the normalized derivative (or logarithm) of the infrared intensity is a constant. This constant is indicative of the partial oxygenation ($S_aO_2$) of the hemoglobin in the arterial blood flow. It is obvious that this ratio changes as $S_aO_2$ changes but, for a short interval with rapid enough sampling rate, the ratio remains constant.

The actual analog data received by the probe interface circuit 102 can include a fairly significant noise component which is caused by a number of sources including motion of the finger 105, the introduction of ambient light into the housing 104, and various sources of electrical noise. These noise components skew the values of either or both of the magnitudes measured in each set of data points destroying the correct relationship between the red and infrared signals. Existing pulse oximeter circuits make use of various filtering techniques to minimize the impact of the noise on the $S_aO_2$ value measured by the system. This filtering circuitry and software or algorithms are analogous to that used in the arterial blood monitoring system 100 and is therefore not described in detail herein.

## System Implementation

Figure 1 illustrates in block diagram form the various components used within the arterial blood monitoring system 100 of the present invention. This embodiment illustrates the use of a plurality of wavelengths of light and, in particular, two wavelengths of light to measure total hemoglobin concentration in the arterial blood. Emitter driver circuit 131 produces the analog drive signals to activate light emitting devices 111, 112 in probe 101. These analog drive signals are carried over cable 103 to probe 101. To measure the concentration of total hemoglobin (tHb) in arterial blood the concentration of several dominant absorbers contained in the arterial blood must be measured. In particular, the concentration of the water and hemoglobin components of the arterial blood must be measured. In order to accomplish this, light emitting device 111 is selected to produce a beam of light at approximately 810nm, which wavelength is substantially isobestic to the oxygenated and deoxygenated components of the hemoglobin in the arterial blood (that is, the extinction coefficients of the oxygenated and deoxygenated hemoglobin are substantially identical). Light emitting device 112 is selected to produce a beam of light at approximately 1270nm. The selection of these two wavelengths is such that water is transparent at the first wavelength of light (810nm) but detected at the second (longer) wavelength of light (1270nm). In addition, these wavelengths are such that the extinction coefficients of the two components (water and hemoglobin) differ at the first wavelength of light. Further, at both wavelengths the two species of hemoglobin are substantially isobestic in extinction but not transparent.

The analog data signals produced by light detector 113 in response to the received beams of light are received from probe 101 over conductors 103 and filtered by analog hardware 132 - 134 in probe interface circuit 102. The input analog data from probe 101 may be decomposed into its non-pulsatile and pulsatile sub-elements in probe interface circuit 102 in order to provide accurate, high resolution, measurements of these components. The pulsatile component typically represents anywhere from .05% to 20% of the total input signal and the decomposition of the input signal into pulsatile and non-pulsatile components permits accurate analog to digital conversion of even the smallest of these pulsatile components.

## Probe Interface Circuit

Probe interface circuit 102 includes emitter driver circuit 131 that is capable of driving light emitting devices 111, 112 such that the light beams produced traverse finger 105 and sufficient light intensity is incident on light detector 113 to produce data indicative of the light absorption of the dominant absorbers in arterial blood. The data produced by light detector 113 (voltage equivalent of the received light intensities) at each wavelength is kept distinct and can be processed independently. This can be done by any of the many schemes presently in use for pulse oximetry, such as time division multiplexing, or frequency division multiplexing.

The light received from finger 105 is converted to an equivalent current signal by the photodiodes of light detector 113, and then converted to a voltage signal by the current to voltage converter 132. The data is then amplified by amplifier 133, and demultiplexed via synchronous demodulation circuit 134. The demultiplexed data comprises analog voltage signals applied to leads CHAN 1, CHAN 2 . . . CHAN n representative of the intensity of the received light at each of the wavelengths of light produced by light emitting devices 111, 112, respectively. The voltage signals on leads CHAN 1, CHAN 2 are then scaled (further amplification) by scaling amplifiers 135 such that they can be converted, with optimal resolution, to a digital equivalent. All channels output by scaling amplifiers 135 are then simultaneously sampled by the sample/hold circuitry 136a, 136b, . . . 136n. The sampled data is passed a channel at a time via multiplexer 137 to the analog to digital converter 138. From there the data, now in digital form, is sent on to data processing circuit 107 where it is stored in memory 106 for processing. The digital data represents the substantially simultaneously sampled amplitudes of the received light intensities from each of the wavelengths used at a sampling frequency of typically 30 Hz or greater. These data values are referred to as $I_1$, $I_2$,....$I_N$, where the subscript indicates the given wavelength. $I_n$ then indicates the received light intensity at any given wavelength.

## Data Processing Circuit

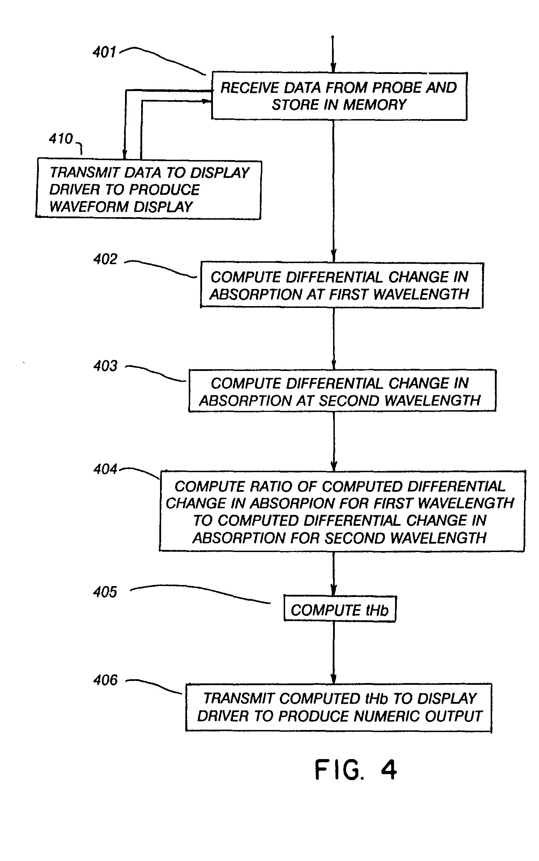

In a two wavelength system, data processing circuit 107 computes a ratio from the digital amplitude data measured at each wavelength of light. In particular, this process used by data processing circuit 107 is illustrated in flow diagram form in Figure 4. At step 401, data processing circuit 107 receives a set of digital input data indicative of the measured intensity of light at both wavelengths, as received by light detector 113. Data processing circuit 107 at step 410 transmits the received set of data to display driver 109 for display in graphical form on display 114. The displayed waveform represents the pulsatile component of the arterial blood. Data processing circuit 107 also stores the received set of data in memory 106 and uses this set of data and the last most recently received set of data to compute at steps 402 and 403 the differential change in absorption of the arterial blood in finger 105 at the first and second selected wavelengths of light, respectively. The differential change in absorption at wavelength n is computed by data processing circuit 107 as:

$$dA_n = \frac{dI_n}{I_n} \qquad\qquad (1)$$

Because $dI_n$ is a mathematical construct, it is approximated in arterial blood monitoring system 100 by $\Delta I_n$, where $\Delta I_n$ is the difference between two consecutively received $I_n$ values. Only $\Delta I$ values that are caused by a small but non zero change in path length through finger 105 are used and therefore $\Delta I_n$ can also be a longer interval of time if necessary to obtain a sufficient change in received intensity of the beam of light. The $I_n$ value used in equation 1 is the average of the two successively received $I_n$ values used to compute $\Delta I_n$.

In a two wavelength system, a final ratio is then calculated by data processing circuit 107 at step 404 as:

$$R = \frac{dA_1}{dA_2} \qquad\qquad (2)$$

where the data values used to compute $dA_1$ are from the same sets of data as the data values used to compute $dA_2$.

This ratio is then used in a calibration equation by data processing circuit 107 at step 405 to relate the R value to a specific total hemoglobin value, which is approximated by a second order polynomial of the form:

$$tHb = AR^2 + BR + C \qquad (3)$$

Where A, B, and C are constants that depend on the specific wavelengths of light used.

The tHb value is then output by data processing circuit 107 at step 406 to display driver 109 (and/or hardcopy) to display in human-readable form on display 115 a numeric value of the concentration of total hemoglobin in the arterial blood of finger 105. Processing then returns to step 401.

## Theory

This device is based on the theory that:

$$dA_n^s = \epsilon_n^s C^s dL^s \qquad (4)$$

A differential change in absorption at a given wavelength n to a given substance ($dA_n^s$), is equal to the extinction of that substance ($\epsilon_n^s$) times the concentration ($C^s$) of that substance times the differential change in pathlength of that substance ($dL^s$).

Further the differential change in absorption can be defined as:

$$dA_n = \frac{dI_n}{I_n} \qquad (5)$$

Note that no measurement of the incident light intensity, Io, is required to measure the differential change in absorption dA. However, samples of $I_n$ must be taken sufficiently close in time so that $\Delta I_n$ represents a good mathematical approximation of $dI_n$.

To determine the relative proportions of two dominant absorbers, in this case water and hemoglobin, one chooses two wavelengths of light at which the two absorbers have extinctions, such that the following set of simultaneous equations has a unique solution for all possible concentrations and pathlengths of the two absorbers.

$$dA_1 = \epsilon_1^t dL^t + \epsilon_1^w dL^w \qquad (6)$$

$$dA_2 = \epsilon_2^t dL^t + \epsilon_2^w dL^w \qquad (7)$$

In this system of equations it is assumed that the only components which change in pathlength are those of the arterial blood. Further it is assumed that the primary absorbers are those of water and hemoglobin where the hemoglobin species in the blood are essentially only those of oxyhemoglobin and reduced hemoglobin. Choosing a wavelength of light that represents an isobestic point for the two species of hemoglobin, such as 804nm, minimizes the effects of oxygen saturation on the tHb readings of the system. The concentration term can be taken as 1 (or 100%) by viewing the optical system as compartmentalized, that is looking at the tissue under test as one in which the light first passes through 100% skin tissue, followed by 100% venous blood, followed by 100% arterial hemoglobin, followed by 100% water, and so on.

In the system of equations (Equations 6, 7) the extinction $\epsilon$ values are constants and it is the job of the arterial blood monitoring system 100 to read the differential change in absorption (dA values) as accurately as possible. This leaves only the values of the differential path lengths dL as unknowns. With two equations, the dL values can be uniquely and simply solved for.

Writing the proportion of hemoglobin in the arterial blood as:

$$Proportion\ Hb = \frac{dL^t}{dL^t + dL^w} \qquad (8)$$

While this proportion is not directly the tHb, it is directly related to it. And while this relationship could be theoretically derived, an empirical relationship (as defined in equation 3) is measured instead. This is necessary due to several ways in which the true optical system of living tissues and realistic optical elements deviate from the exact theoretical model developed here. Equation 3 is therefore referred to as the calibration equation and its coefficients A, B, and C, are experimentally derived via clinical testing. The coefficients are then installed in the arterial blood monitoring system software. It should be noted that these coefficients differ for different wavelength emitters.

The wavelengths of light produced by light emitting devices 111, 112 are also selected so as to optimize the performance of the entire electro optical system: low enough light absorption so that sufficient optical signal is received by light detector 113 and high enough light absorption so that there is an appreciable change in light absorption over the range of physiological changes in pathlength caused by the pulsation of arterial blood. Typical wavelengths of light selected for a realization of this system are 810nm and 1270nm, however many wavelength combinations meeting the above criteria can be used.

## Combination tHb Monitor and Pulse Oximeter

The methodologies for pulse oximetry are well known. The method of obtaining tHb noninvasively and in real time has been disclosed above. The arterial blood monitoring system of the present invention combines the two technologies to create an improved device for measurement of both parameters. tHb is an interfering substance in the measurement of $S_aO_2$ by the present technologies. By "interfering substance" it is meant that variations in tHb cause variations in the $S_aO_2$ as read by a pulse oximeter. These variations in $S_aO_2$ are correlated to, but not corrected for, the tHb level. A device capable of measuring tHb can therefore provide a means for eliminating the error it causes in determining $S_aO_2$. The same holds true in terms of $S_aO_2$ being an interfering substance in the measurement of tHb. The solution to this problem lies in a combination device capable of reading both parameters. Such a device can be simply obtained by using two wavelengths to derive the $S_aO_2$, and two more as described above for obtaining tHb. The resulting values for $S_aO_2$ and tHb can then be used to correct the readings of the other. A more sophisticated system uses a three wavelength system, where the practical realization of this system utilizes the standard oximetry wavelengths of 660nm and 940nm produced by two light emitting devices 111a, 111b, along with a wavelength of 1270nm produced by a light emitting device 112. (Once again, any three wavelengths that meet the criteria stated above for a standalone tHb system can be used.) In addition, the two segment light detector 113 is activated in a manner to reflect the use of three wavelengths of light. Silicon photodiode 113a detects both of the light beams (660nm, 940nm) produced by light emitter devices 111a, 111b and its output is demultiplexed to separate the two measured values of light intensity. Germanium photodiode 113b of light detector 113 measures the intensity of the third beam of light at 1270nm.

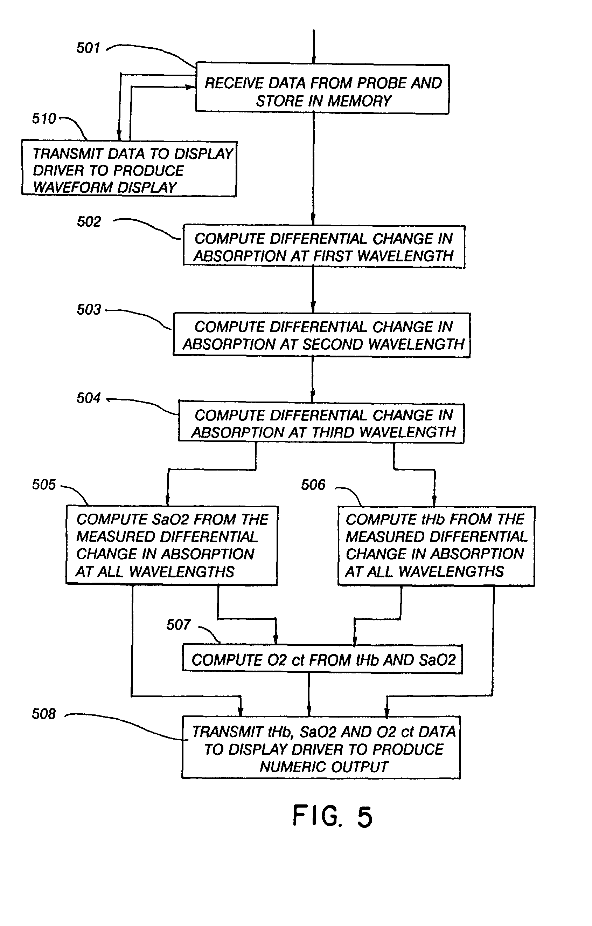

In particular, the process used by data processing circuit 107 is illustrated in flow diagram form in Figure 5. At step 501, data processing circuit 107 receives a set of digital input data indicative of the measured intensity of light at all three wavelengths, as received by light detector 113. Data processing circuit 107 at step 510 transmits the received set of data to display driver 109 for display in graphical form on display 114. The displayed waveform represents the pulsatile component of the arterial blood. Data processing circuit 107 also stores the received set of data in memory 101 and uses this set of data and the last most recently received set of data to compute at steps 502 - 504 the differential change in absorption of the arterial blood in finger 105 at the first, second, and third wavelengths of light, respectively.

Thus, as noted above, and extrapolated to a three variable system:

$$dA\lambda = \epsilon_\lambda^O dL^O + \epsilon_\lambda^R dL^R + \epsilon_\lambda^W dL^W \qquad (9)$$

where

$O = [O_2Hb]$
$R = [RHb]$
$W = [H_2O]$

Therefore at any given wavelength, $\lambda$, the differential change in light absorption is a function of the change in path length of the three absorbers: oxyhemoglobin ($O_2Hb$), reduced hemoglobin (RHb), and water ($H_2O$).

$$tHb \propto \frac{dL^O + dL^R}{dL^O + dL^R + dL^W} \qquad (10)$$

Note that this equation shows only that the total hemoglobin is proportional to this ratio of path length changes, not equal to it. This is due, at least in part, to the fact the tHb is measured in terms of grams/deciliter of whole blood and this equation is a ratio of path lengths. There is a one to one correspondence between this ratio of path lengths and the tHb which is determined, and curve fit, experimentally. The empirical curve fit also compensates for the differences between the theoretical models and the actual optical systems.

$$S_a O_2 = \frac{dL^O}{dL^O + dL^R} \qquad (11)$$

For a three wavelength system, with the subscripts 1, 2, and 3 indicating the specific wavelengths used we can write the following system of equations

$$dA_1 = \epsilon_1^O \, dL^O + \epsilon_1^R \, dL^R + \epsilon_1^W \, dL^W \qquad (12)$$

$$dA_2 = \epsilon_2^O \, dL_O + \epsilon_2^R \, dL^R + \epsilon_2^W \, dL^W \qquad (13)$$

$$dA_3 = \epsilon_3^O \, dL^O + \epsilon_3^R \, dL^R + \epsilon_3^W \, dL^W \qquad (14)$$

In matrix notation:

$$\begin{pmatrix} dA_1 \\ dA_2 \\ dA_3 \end{pmatrix} = \begin{pmatrix} dL^O \\ dL^R \\ dL^W \end{pmatrix} \begin{pmatrix} \epsilon_1^O & \epsilon_1^R & \epsilon_1^W \\ \epsilon_2^O & \epsilon_2^R & \epsilon_2^W \\ \epsilon_3^O & \epsilon_3^R & \epsilon_3^W \end{pmatrix} \qquad (15)$$

This allows us to solve for the path length contributions of each of the three absorbers as defined by the following equations

$$dL^O = \frac{dA_1(\epsilon_2^R\epsilon_3^Y - \epsilon_3^R\epsilon_2^Y) - dA_2(\epsilon_1^R\epsilon_3^Y - \epsilon_3^R\epsilon_1^Y) + dA_3(\epsilon_1^R\epsilon_2^Y - \epsilon_2^R\epsilon_1^Y)}{\Delta} \qquad (16)$$

$$dL^R = \frac{\epsilon_1^O(dA_2\epsilon_3^Y - dA_3\epsilon_2^Y) - \epsilon_2^O(dA_1\epsilon_3^Y - dA_3\epsilon_1^Y) + \epsilon_3^O(dA_1\epsilon_2^Y - dA_2\epsilon_1^Y)}{\Delta} \qquad (17)$$

$$dL^Y = \frac{\epsilon_1^O(\epsilon_2^R dA_3 - \epsilon_3^R dA_2) - \epsilon_2^O(\epsilon_1^R dA_3 - \epsilon_3^R dA_1) + \epsilon_3^O(\epsilon_1^R dA_2 - \epsilon_2^R dA_1)}{\Delta} \qquad (18)$$

Now the $S_aO_2$ can be calculated by data processing circuit 107 at step 405 as

$$S_aO_2 = \frac{dL^O}{dL^O + dL^R} \qquad (19)$$

$$= \frac{[dA_1(\epsilon_2^R\epsilon_3^Y - \epsilon_3^R\epsilon_2^Y) - dA_2(\epsilon_1^R\epsilon_3^Y - \epsilon_3^R\epsilon_1^Y) + dA_3(\epsilon_1^R\epsilon_2^Y - \epsilon_2^R\epsilon_1^Y)]/\Delta}{[dA_1(\epsilon_2^R\epsilon_3^Y - \epsilon_3^R\epsilon_2^Y) - dA_2(\epsilon_1^R\epsilon_3^Y - \epsilon_3^R\epsilon_1^Y) + dA_3(\epsilon_1^R\epsilon_2^Y - \epsilon_2^R\epsilon_1^Y) + \epsilon_1^O(dA_2\epsilon_3^Y - dA_3\epsilon_2^Y) - \epsilon_2^O(dA_1\epsilon_3^Y - dA_3\epsilon_1^Y) + \epsilon_3^O(dA_1\epsilon_2^Y - dA_2\epsilon_1^Y)]/\Delta} \qquad (20)$$

$$= \frac{dA_1(\epsilon_2^R\epsilon_3^Y - \epsilon_2^Y\epsilon_3^R) - dA_2(\epsilon_1^R\epsilon_3^Y - \epsilon_1^Y\epsilon_3^R) + dA_3(\epsilon_1^R\epsilon_2^Y - \epsilon_1^Y\epsilon_2^R)}{dA_1(\epsilon_2^R\epsilon_3^Y - \epsilon_2^Y\epsilon_3^R - \epsilon_2^O\epsilon_3^Y + \epsilon_2^Y\epsilon_3^O) + dA_2(\epsilon_1^Y\epsilon_3^R - \epsilon_1^R\epsilon_3^Y + \epsilon_1^O\epsilon_3^Y - \epsilon_1^Y\epsilon_3^O) + dA_3(\epsilon_1^R\epsilon_2^Y - \epsilon_2^R\epsilon_1^Y - \epsilon_1^O\epsilon_2^Y + \epsilon_2^O\epsilon_1^Y)} \qquad (21)$$

Recognizing that the extinction coefficients are empirically measured constants, using $K_1$, through $K_6$ to indicate the appropriate combination of extinction coefficients, we can simplify the equation for $S_aO_2$ as follows

$$S_aO_2 = \frac{dA_1(K_1) + dA_2(K_2) + dA_3(K_3)}{dA_1(K_4) + dA_2(K_5) + dA_3(K_6)} \qquad (22)$$

The same development that was performed for $S_aO_2$ can now be done by data processing circuit 107 at step 406 for total hemoglobin. Notice that the numerator in this equation is identical to the denominator in the equation for $S_aO_2$.

$$tHb \propto \frac{dL^O + dL^R}{dL^W + dL^O + dL^R} \qquad (23)$$

$$tHb \propto \frac{\begin{array}{c} dA_1 \, (\epsilon_2^R \epsilon_3^W - \epsilon_2^W \epsilon_3^R - \epsilon_2^O \epsilon_3^W + \epsilon_2^W \epsilon_3^O) + dA_2 \, (\epsilon_1^W \epsilon_3^R - \epsilon_1^R \epsilon_3^W + \epsilon_1^O \epsilon_3^W - \epsilon_1^W \epsilon_3^O) + \\ dA_3 \, (\epsilon_1^R \epsilon_2^W - \epsilon_2^R \epsilon_1^W + \epsilon_2^O \epsilon_1^W - \epsilon_1^O \epsilon_2^W) \end{array}}{\begin{array}{c} dA_1 \, (\epsilon_2^O \epsilon_3^R - \epsilon_2^R \epsilon_3^O + \epsilon_2^W \epsilon_3^O - \epsilon_2^O \epsilon_3^W + \epsilon_2^R \epsilon_3^W - \epsilon_3^W \epsilon_2^R) + \\ dA_2 \, (\epsilon_1^R \epsilon_3^O - \epsilon_1^O \epsilon_3^R + \epsilon_1^O \epsilon_3^W - \epsilon_1^W \epsilon_3^O + \epsilon_1^W \epsilon_3^R - \epsilon_1^R \epsilon_3^W) + \\ dA_3 \, (\epsilon_1^O \epsilon_2^R - \epsilon_1^R \epsilon_2^O + \epsilon_2^O \epsilon_1^W - \epsilon_2^W \epsilon_1^O + \epsilon_1^R \epsilon_2^W - \epsilon_1^W \epsilon_2^R) \end{array}}$$

$$(24)$$

$$tHb \propto \frac{dA_1 \, (K_4) + dA_2 \, (K_5) + dA_3 \, (K_6)}{dA_1 \, (K_7) + dA_2 \, (K_8) + dA_3 \, (K_9)} \qquad (25)$$

### Oxygen Content

With tHb and $S_aO_2$ known it is a simple matter to calculate and display the O2 content of the arterial blood. This is derived by data processing circuit 107 at step 407 as follows:

$$O2 \; ct = (.0031 * PO_2) + (1.38 * tHb * s_aO_2) \qquad (26)$$

tHb and $S_aO_2$ are the analytes measured by the arterial blood monitoring system and $Po_2$ can either be taken as a fixed value of 90 torr or for increased accuracy can be obtained by working backwards through the oxygen dissociation curve. The curve can be installed as a look-up table in memory 107 or an equation in the software of data processing circuit 107. As the pH, 2,3 DPG and $PaCO_2$ are unknown, the curve used assumes an average or normal level for these variables. Since the dissolved oxygen is such a small contribution to the total O2 ct, either of these methods provides adequate accuracy. At step 508, data processing circuit 107 transmits the computed values of $S_aO_2$, tHb and O2 ct to display driver 109 to produce numerical human-readable outputs on display devices 115a, 115b, 115c, respectively.

There are several different methodologies that accomplish the same purpose. One of these is a logarithmically based method. In this method, $R_n$ as defined in equation a is modified as follows:

$$R_n = \frac{\log I_{n(systole)}}{\log I_{n(diastole)}} \qquad (27)$$

The log values are to the base e. Systole and diastole refer to the two points on the photoplethysmographic waveform where the transmitted intensity is sampled.

These log values can be calculated (or obtained via lookup table in memory 106) by data processing circuit 107 using the circuitry already defined. Alternatively the circuitry can be altered to utilize logarithmic amplifiers so that the data sampled by data processing circuit 107 is already converted to logarithms or is the final ratio calculated in equation 2. One advantage of this methodology is that it allows one to work with the max and min values of the photoplethysmographic waveform. It is worth noting that the sample points on the photopethysmographic waveform do not necessarily have to be at systole and diastole. They can be any two points separated by a measurable change in arterial path length.

While a specific embodiment of this invention has been disclosed, it is expected that those skilled in the art can and will design alternate embodiments of this invention that fall within the scope of the appended claims.

## Claims

1. Apparatus for noninvasively measuring a concentration of total hemoglobin in arterial blood, which includes first and second dominant absorbers, in a subject comprising:

    probe means which noninvasively produces data indicative of a change in light absorption as a function of a change in path length in said arterial blood in said subject at first and second predetermined light wavelengths, wherein said first and second dominant absorbers have different extinction coefficients at at least one of said first and second light wavelengths;

    wherein said first and second dominant absorbers are water and hemoglobin, which contain oxygenated and deoxygenated components; and

    means responsive to said data, for producing an indication of total hemoglobin concentration in said arterial blood.

2. Apparatus for noninvasively measuring a concentration of total hemoglobin in arterial blood, which includes first and second dominant absorbers, in a subject comprising:

    probe means which noninvasively produces data indicative of a change in light absorption as a function of a change in path length in said arterial blood in said subject at first and second predetermined light wavelengths, wherein said first and second dominant absorbers have different extinction coefficients at at least one of said first and second light wavelengths, including:

    means for generating a first beam of light at said first predetermined light wavelength,

    means for generating a second beam of light at said second predetermined light wavelength,

    means for applying said first and said second beams of light to said arterial blood in said subject,

    means for measuring a change in light absorption of said first and second beams of light indicative of said change in arterial blood path length in said subject;

    wherein said first and second dominant absorbers are water and hemoglobin, which contain oxygenated and deoxygenated components;

    means, responsive to said data, for producing an indication of total hemoglobin concentration in said arterial blood, including:

    means, responsive to said data, for computing a numerical relationship between said measured change in light absorption of said first and said second beams of light in said arterial blood in said subject,

    means for defining a correspondence between said computed numerical relationship and said hemoglobin concentration,

    means, responsive to said defining means and said computed numerical relationship, for determining said total hemoglobin concentration;

    means, responsive to said producing means, for displaying in human readable form said indication of total hemoglobin concentration in said arterial blood.

3. A method for noninvasively measuring a concentration of total hemoglobin in arterial blood, which includes first and second dominant absorbers, in a subject comprising the steps of:

    noninvasively producing data indicative of a change in light absorption as a function of a change in path length in said arterial blood in said subject at first and second predetermined light wavelengths, wherein said first and second dominant absorbers have different extinction coefficients at at least one of said first and second light wavelengths;

    wherein said first and second dominant absorbers are water and hemoglobin, which contain oxygenated and deoxygenated components; and

    producing an indication of total hemoglobin concentration in said arterial blood.

4. A method for noninvasively measuring a concentration of total hemoglobin in arterial blood, which includes first and second dominant absorbers, in a subject comprising the steps of:

    noninvasively producing data indicative of a change in light absorption as a function of a change in path length in said arterial blood in said subject at first and second predetermined light wavelengths, wherein said first and second dominant absorbers have different extinction coefficients at at least one of said first and second light wavelengths, including:

generating a first beam of light at said first predetermined light wavelength,

generating a second beam of light at said second predetermined light wavelength,

applying said first and said second beams of light to said arterial blood in said subject,

measuring a change in light absorption of said first and second beams of light indicative of said change in arterial blood path length in said subject;

wherein said first and second dominant absorbers are water and hemoglobin, which contain oxygenated and deoxygenated components;

producing, in response to said data, an indication of total hemoglobin concentration in said arterial blood, including:

computing a numerical relationship between said measured change in light absorption of said first and said second beams of light in said arterial blood in said subject,

defining a correspondence between said computed numerical relationship and said hemoglobin concentration,

determining, in response to said step of defining and said computed numerical relationship, said total hemoglobin concentration;

displaying in human readable form said indication of total hemoglobin concentration in said arterial blood.

5. Apparatus for noninvasively measuring a concentration of total hemoglobin in arterial blood, which includes a plurality of dominant absorbers, in a subject comprising:

probe means, which noninvasively produces data indicative of a change in light absorption as a function of a change in path length in said arterial blood in said subject at a plurality of predetermined light wavelengths, wherein a first and a second dominant absorber of said plurality of dominant absorbers have different extinction coefficients at at least one of a first and a second of said plurality of light wavelengths;

wherein said first and second dominant absorbers include water and hemoglobin, which contain at least first and second components; and

means responsive to said data for producing an indication of total hemoglobin concentration in said arterial blood.

6. Apparatus for noninvasively measuring a concentration of total hemoglobin in arterial blood, which includes a plurality of dominant absorbers, in a subject comprising:

probe means, which noninvasively produces data indicative of a change in light absorption as a function of a change in path length in said arterial blood in said subject at a plurality of predetermined light wavelengths, wherein a first and a second dominant absorber of said plurality of dominant absorbers have different extinction coefficients at at least one of a first and a second of said plurality of light wavelengths, including:

means for generating a first beam of light at said first predetermined light wavelength,

means for generating a second beam of light at said second predetermined light wavelength,

means for applying said first and said second beams of light to said arterial blood in said subject,

means for measuring said change in light absorption of said first and second beams of light indicative of said change in arterial blood path length in said subject;

wherein said first and second dominant absorbers include water and hemoglobin, which contain at least first and second components;

means responsive to said data for producing an indication of total hemoglobin concentration in said arterial blood, including:

means responsive to said data for computing a numerical relationship between said measured change in light absorption of said first and said second beams of light in said arterial blood in said subject,

means for defining a correspondence between said computed numerical relationship and said hemoglobin concentration,

means, responsive to said defining means and said computed numerical relationship, for determining said total hemoglobin concentration;

wherein said probe means further includes:

means for generating a third of said plurality of light wavelengths, selected to be absorbed by one of said first and second components at an absorption rate different than at said first and second wavelengths,

means for applying said third beam of light to said arterial blood in said subject,

means for measuring said change in light absorption of said third beam of light indicative of said change in arterial blood path length in said subject;

means, responsive to a measured change in light absorption indicative of said change in arterial blood path length in said subject at said third light wavelength and said determined total hemoglobin concentration, for computing oxygen saturation of said arterial blood in said subject;

means, responsive to said producing means, for displaying in human readable form said indication of total hemoglobin concentration in said arterial blood.

7. A method for noninvasively measuring a concentration of total hemoglobin in arterial blood, which includes a plurality of dominant absorbers, in a subject comprising the steps of:

noninvasively producing data indicative of a change in light absorption as a function of a change in path length in said arterial blood in said subject at a plurality of predetermined light wavelengths, wherein a first and a second dominant absorber of said plurality of dominant absorbers have different extinction coefficients at at least one of a first and a second of said plurality of light wavelengths;

wherein said first and second dominant absorbers include water and hemoglobin, which contain at least first and second components; and

producing an indication of total hemoglobin concentration in said arterial blood.

8. A method of noninvasively measuring a concentration of total hemoglobin in arterial blood, which includes a plurality of dominant absorbers, in a subject comprising the steps of:

noninvasively producing data indicative of a change in light absorption as a function of a change in path length in said arterial blood in said subject at a plurality of predetermined light wavelengths, wherein a first and a second dominant absorber of said plurality of dominant absorbers have different extinction coefficients at at least one of a first and a second of said plurality of light wavelengths, including:

generating a first beam of light at said first predetermined light wavelength,

generating a second beam of light at said second predetermined light wavelength,

applying said first and said second beams of light to said arterial blood in said subject,

measuring said change in light absorption of said first and second beams of light indicative of said change in arterial blood path length in said subject;

wherein said first and second dominant absorbers include water and hemoglobin, which contain at least first and second components;

producing, in response to said data, in indication of total hemoglobin concentration in said arterial blood, including:

computing a numerical relationship between said measured change in light absorption of said first and said second beams of light in said arterial blood in said subject,

defining a correspondence between said computed numerical relationship and said hemoglobin concentration,

determining, in response to said step of defining and said computed numerical relationship, said total hemoglobin concentration;

computing, in response to a measured change in light absorption indicative of said change in arterial blood path length in said subject at a third of said plurality of light wavelengths, wherein said third wavelength is selected to be absorbed by one of said first and second components at an absorption rate different than at said first and second wavelengths, and said determined total hemoglobin concentration, oxygen saturation of said arterial blood in said subject;

displaying in human readable form said indication of total hemoglobin concentration in said arterial blood.

9. Apparatus for noninvasively measuring a concentration of total hemoglobin in arterial blood, which includes first and second dominant absorbers, in a subject comprising:

means for generating a first beam of light at a first predetermined light wavelength;

means for generating a second beam of light at a second predetermined light wavelength, wherein said first and second dominant absorbers have different extinction coefficients at at least one of said first and second light wavelengths;

means for noninvasively applying said first and said second beams of light to said arterial blood in said subject;

means for measuring a change in light absorption of said first and second beams of light by said arterial blood indicative of a change in path length in said arterial blood in said subject;

wherein said first and second dominant absorbers are water and hemoglobin, which contain oxygenated and deoxygenated components; and

means responsive to said measured change in path length in said arterial blood in said subject for producing an indication of total hemoglobin concentration in said arterial blood.

10. Apparatus for noninvasively measuring a concentration of total hemoglobin in arterial blood, which includes first and second dominant absorbers, in a subject comprising:

means for generating a first beam of light at a first predetermined light wavelength;

means for generating a second beam of light at a second predetermined light wavelength, wherein said first and second dominant absorbers have different extinction coefficients at at least one of said first and second light wavelengths;

means for noninvasively applying said first and said second beams of light to said arterial blood in said subject;

means for measuring a change in light absorption of said first and second beams of light by said arterial blood indicative of a change in path length in said arterial blood in said subject;

wherein said first and second dominant absorbers are water and hemoglobin, which contain oxygenated and deoxygenated components; and

means responsive to said measured change in path length in said arterial blood in said subject for producing an indication of total hemoglobin concentration in said arterial blood, including:

means responsive to said measured change in path length in said arterial blood in said subject for computing a numerical relationship between said measured change in light absorption of said first and said second beams of light in said arterial blood in said subject,

means for defining a correspondence between said computed numerical relationship and said hemoglobin concentration; and

means, responsive to said defining means and said computed numerical relationship, for determining said total hemoglobin concentration;

means for generating a third beam of light at a third light wavelength which is selected to be absorbed by one of said first and second components at an absorption rate different than at said first and second wavelengths;

means for noninvasively applying said third beam of light to said arterial blood in said subject;

means for measuring a change in light absorption of said third beam of light by said arterial blood indicative of a change in path length in said arterial blood in said subject; and

means, responsive to said measured change in light absorption at said third light wavelength and said determined total hemoglobin concentration, for computing oxygen saturation of said arterial blood in said subject.

11. A method for noninvasively measuring a concentration of total hemoglobin in arterial blood, which includes first and second dominant absorbers, in a subject comprising the steps of:

generating a first beam of light at a first predetermined light wavelength;

generating a second beam of light at a second predetermined light wavelength, wherein said first and second dominant absorbers have different extinction coefficients at at least one of said first and second light wavelengths;

noninvasively applying said first and said second beams of light to said arterial blood in said subject;

measuring a change in light absorption of said first and second beams of light by said arterial blood indicative of a change in path length in said arterial blood in said subject;

wherein said first and second dominant absorbers are water and hemoglobin, which contain oxygenated and deoxygenated components; and

producing, in response to said measured change in path length in said arterial blood in said subject, an indication of total hemoglobin concentration in said arterial blood.

12. A method for noninvasively measuring a concentration of total hemoglobin in arterial blood, which includes first and second dominant absorbers, in a subject Comprising the steps of:

generating a first beam of light at a first predetermined light wavelength;

generating a second beam of light at a second predetermined light wavelength, wherein said first and second dominant absorbers have different extinction coefficients at at least one of said first and second light wavelengths;

noninvasively applying said first and said second beams of light to said arterial blood in said subject;

measuring a change in light absorption of said first and second beams of light by said arterial blood indicative of a change in path length in said arterial blood in said subject;

wherein said first and second dominant absorbers are water and hemoglobin, which contain oxygenated and deoxygenated components; and

producing, in response to said measured change in path length in said arterial blood in said subject, an indication of total hemoglobin concentration in said arterial blood, including:

computing a numerical relationship between said measured change in light absorption of said first and said second beams of light in said arterial blood in said subject,

defining a correspondence between said computed numerical relationship and said hemoglobin concentration; and

determining, in response to said step of defining and said computed numerical relationship, said total hemoglobin concentration;

generating a third beam of light at a third light wavelength which is selected to be absorbed by one of said first and second components at an absorption rate different than at said first and second wavelengths;

noninvasively applying said third beam of light to said arterial blood in said subject;

measuring a change in light absorption of said third beam of light by said arterial blood indicative of a change in path length in said arterial blood in said subject; and

computing, in response to said measured change in light absorption at said third light wavelength and said determined total hemoglobin concentration, oxygen saturation of said arterial blood in said subject.

FIG. 1

**DISPLAY MODULE**

115a
115b

PLETHYSMOGRAPHIC WAVEFORM

95 — SaO2

19.2 — TOTAL HEMOGLOBIN (g/dl)

14.5 — O2 ct (VOL %)

114

115c

FIG. 2

FIG. 3

ARTERIAL PULSATION ABSORPTION

ARTERIAL BLOOD ABSORPTION

VENOUS BLOOD ABSORPTION

OTHER TISSUE ABSORPTION

ABSORPTION

TIME

18

401 → RECEIVE DATA FROM PROBE AND STORE IN MEMORY

410 → TRANSMIT DATA TO DISPLAY DRIVER TO PRODUCE WAVEFORM DISPLAY

402 → COMPUTE DIFFERENTIAL CHANGE IN ABSORPTION AT FIRST WAVELENGTH

403 → COMPUTE DIFFERENTIAL CHANGE IN ABSORPTION AT SECOND WAVELENGTH

404 → COMPUTE RATIO OF COMPUTED DIFFERENTIAL CHANGE IN ABSORPION FOR FIRST WAVELENGTH TO COMPUTED DIFFERENTIAL CHANGE IN ABSORPTION FOR SECOND WAVELENGTH

405 → COMPUTE tHb

406 → TRANSMIT COMPUTED tHb TO DISPLAY DRIVER TO PRODUCE NUMERIC OUTPUT

FIG. 4

501 → RECEIVE DATA FROM PROBE AND STORE IN MEMORY

510 → TRANSMIT DATA TO DISPLAY DRIVER TO PRODUCE WAVEFORM DISPLAY

502 → COMPUTE DIFFERENTIAL CHANGE IN ABSORPTION AT FIRST WAVELENGTH

503 → COMPUTE DIFFERENTIAL CHANGE IN ABSORPTION AT SECOND WAVELENGTH

504 → COMPUTE DIFFERENTIAL CHANGE IN ABSORPTION AT THIRD WAVELENGTH

505 → COMPUTE $SaO_2$ FROM THE MEASURED DIFFERENTIAL CHANGE IN ABSORPTION AT ALL WAVELENGTHS

506 → COMPUTE tHb FROM THE MEASURED DIFFERENTIAL CHANGE IN ABSORPTION AT ALL WAVELENGTHS

507 → COMPUTE $O_2$ ct FROM tHb AND $SaO_2$

508 → TRANSMIT tHb, $SaO_2$ AND $O_2$ ct DATA TO DISPLAY DRIVER TO PRODUCE NUMERIC OUTPUT

FIG. 5